# FASCICULE DE BREVET EUROPEEN

(11) **EP 1 120 103 B1**
(45) Date de publication et mention de la délivrance du brevet: **15.12.2004**
(21) Numéro de dépôt: 00403529.1
(22) Date de dépôt: 14.12.2000
(51) Int. Cl.: A61K 7/06

(54) **Compositions cosmétiques contenant un amidon amphotère et un agent conditionneur cationique et leurs utilisations**
Kosmetische Zusammensetzungen, die eine amphotere Stärke und ein kationisches Konditionierungsmittel enthalten, und deren Verwendungen
Cosmetic compositions containing an amphoteric starch and a cationic conditioning agent and uses thereof

(30) Priorité: 13.01.2000 FR 0000409
(43) Date de publication de la demande: 01.08.2001
(73) Titulaire: L'OREAL, 75008 Paris (FR)
(72) Inventeur: Douin, Véronique, 75017 Paris (FR); Chesneau, Laurent, 92300 Levallois Perret (FR); Descoster, Sandrine, 95210 Saint Gratien (FR)
(74) Mandataire: Le Blainvaux Bellegarde, Françoise

(56) Documents cités:
- EP-A- 0 689 829
- EP-A- 0 797 979
- EP-A- 0 948 960
- National Starch: "Structure Solanace" (Online). Retrieved from the internet: <URL: http://www.nationalstarch.com/solan.htm> 23 Octobre 2000 XP002150732

## Description

La présente invention concerne de nouvelles compositions cosmétiques comprenant dans un milieu cosmétiquement acceptable au moins un agent conditionneur cationique particulier et au moins un amidon amphotère particulier.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).
En outre, l'usage des polymères cationiques dans ce but présente divers inconvénients.
En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

En résumé, il s'avère que les compositions cosmétiques actuelles contenant des agents conditionneurs, ne donnent pas complètement satisfaction.

EP-A-0 797 979 divulgue des compositions cosmétiques contenant un amidon amphotère et un polymère fixant pouvant être cationique. EP-A-0 948 960 divulgue des amidons modifiés "nonioniquement" qui sont éventuellement modifiés par des groupements zwittérioniques.

La demanderesse a maintenant découvert que l'association d'un amidon amphotère défini ci-dessous avec certains agents conditionneurs permet de remédier à ces inconvénients.

Ainsi, à la suite d'importantes recherches menées sur la question, il a maintenant été trouvé par la Demanderesse qu'en introduisant un amidon amphotère particulier dans les compositions en particulier capillaires de l'art antérieur à base d'agents conditionneurs, il est possible de limiter, voire supprimer, les problèmes généralement liés à l'emploi de telles compositions, à savoir en particulier l'alourdissement (toucher chargé lors d'applications répétées), le manque de lissage et de souplesse des cheveux, tout en conservant les autres propriétés cosmétiques avantageuses qui sont attachés aux compositions à base d'agents conditionneurs.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, a) au moins un amidon amphotère défini ci-dessous.
et b) au moins un agent conditionneur cationique choisi parmi :
- les polymères de polyammonium quaternaires définis ci-dessous
- les silicones cationiques,
- les tensioactifs de type sels d'ammonium quaternaires, et
- les cyclopolymères d'alkyl diallyl amine ou de dialkyl diallyl ammonium definis ci-dessous.

Un autre objet de l'invention concerne l'utilisation d'un amidon amphotère défini ci-dessous dans, ou pour la fabrication d'une composition cosmétique comprenant un agent conditionneur tel que défini ci-dessus.

Les différents objets de l'invention vont maintenant être détaillés. L'ensemble des significations et définitions des composés utilisés dans la présente invention données ci-dessous sont valables pour l'ensemble des objets de l'invention.

Dans le cadre de la présente demande, on entend par agent conditionneur tout agent ayant pour fonction l'amélioration des propriétés cosmétiques des cheveux, en particulier la douceur, le démêlage, le toucher, l'électricité statique.

Les compositions selon l'invention comprennent nécessairement un amidon amphotère choisi parmi les composés de formules suivantes : 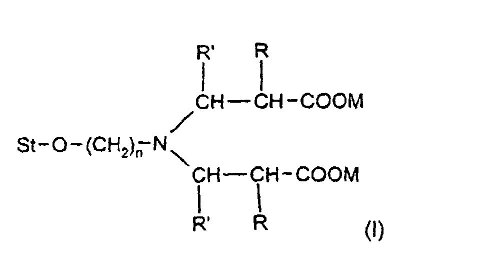 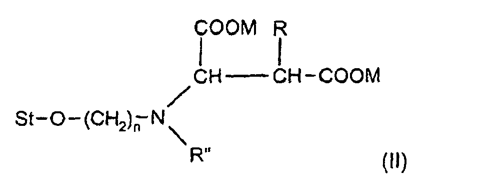 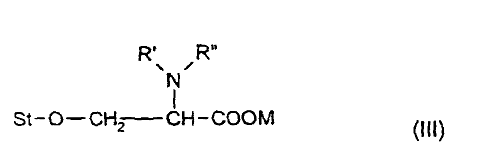 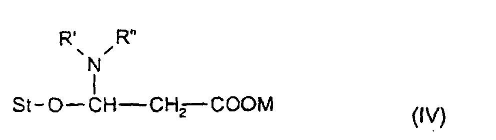 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄ , un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2.

Les amidons amphotères selon l'invention peuvent être utilisées dans les compositions conformes à l'invention dans des concentrations généralement comprises entre 0,01 et 10 %, et de préférence entre 0,1 et 5 % en poids par rapport au poids total de la composition.

Les polymères de polyammonium quaternaires sont choisis parmi :
(1) les polymères de diammonium quaternaires contenant des motifs récurrents répondant à la formule (IV) : 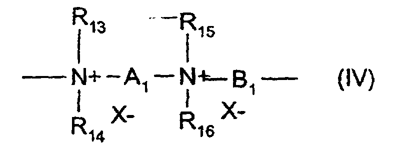 formule (IV) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 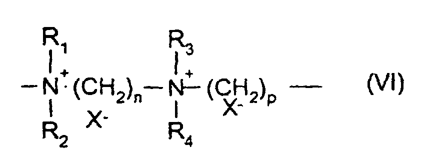 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VI) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(2) les polymères de polyammonium quaternaires constitués de motifs de formule (VII): 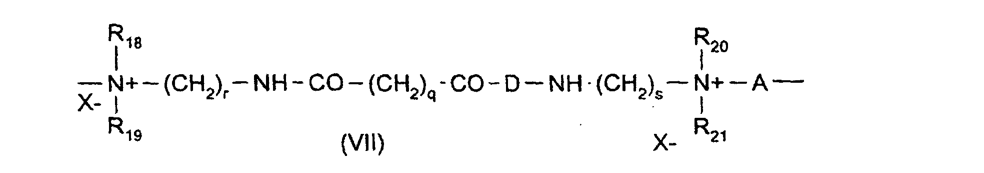 formule dans laquelle:
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion d'un acide minéral ou organique tel qu'un halogènure,
   D peut être nul ou peut représenter un groupement -(CH₂)ₜ-CO- dans lequel t désigne un nombre égal à 4 ou à 7,

A désigne un radical d'un dihalogénure ou représente de préférence -CH₂-CH₂-O-CH₂-CH₂-
De tels composés sont notamment décrits dans la demande de brevet EP-A-122 324.

On peut par exemple citer parmi ceux-ci, les produits "Mirapol® A 15", "Mirapol® AD1", "Mirapol® AZ1" et "Mirapol® 175" vendus par la société Miranol.

Selon l'invention, on désigne par silicone cationique toute silicone comportant au moins une amine primaire, secondaire, tertiaire ou un groupement ammonium quaternaire. On peut ainsi citer :
(a) les polysiloxanes dénommés dans le dictionnaire CTFA "amodiméthicone" et répondant à la formule : 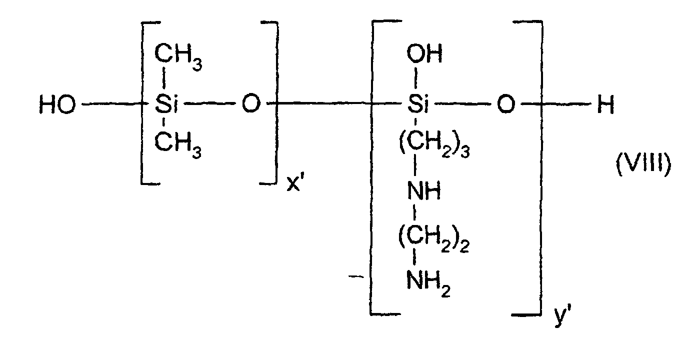 dans laquelle x' et y' sont des nombres entiers dépendant du poids moléculaire, généralement tels que ledit poids moléculaire moyen en poids est compris entre 5 000 et 500 000 environ ;
(b) les silicones aminées répondant à la formule :

   R'ₐG₃₋ₐ-Si(OSiG₂)ₙ-(OSiG_{b}R'_{2-b})ₘ-O-SiG₃₋ₐ-R'ₐ (IX)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :

      -NR"-CH₂CH₂-N'(R")₂

      -N(R")₂

      -N^{⊕}(R")₃ A⁻

      -NH® (R")₂ A⁻

      -NH₂^{⊕}(R")A⁻

      -N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Un produit correspondant à cette définition est la silicone dénommée "triméthylsilylamodiméthicone", répondant à la formule : 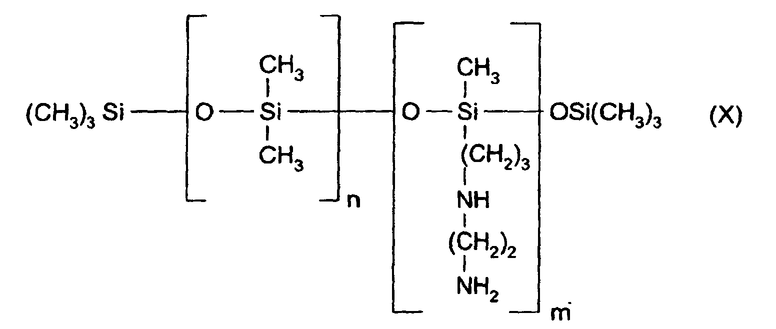 dans laquelle n et m ont les significations données ci-dessus (cf formule IX).
   De tels polymères sont décrits par exemple dans la demande de brevet EP-A-95238.
(c) les silicones aminées répondant à la formule : 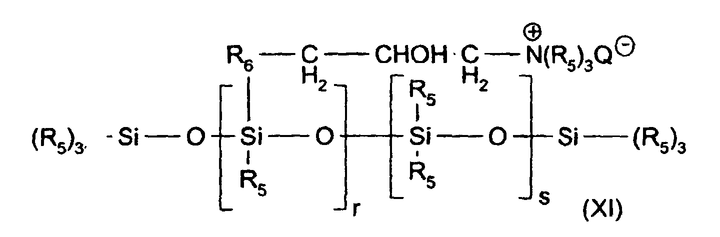 dans laquelle:
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate ...);
   r représente une valeur statistique moyenne de 2 à 20 et en particulier de 2 à 8 ;
   s représente une valeur statistique moyenne de 20 à 200 et en particulier de 20 à 50.

   De tels silicones aminées sont décrites plus particulièrement dans le brevet US 4 185 087.
   Une silicone entrant dans cette classe est la silicone commercialisée par la société Union Carbide sous la dénomination "Ucar Silicone ALE 56.
d) les silicones ammonium quaternaire de formule : 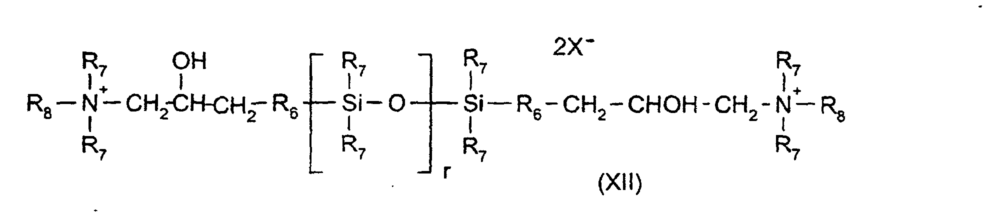 dans laquelle:
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C,₈, un radical alcényle en C₂-C₁₈ , un radical -R₆-NHCOR₇;
   X- est un anion tel qu'un ion halogénure, notamment chlorure ou un sel d'acide organique (acétate ...);
   r représente une valeur statistique moyenne de 2 à 200 et en particulier de 5 à 100 ;

   Ces silicones sont par exemple décrites dans la demande EP-A-0530974.
   Des silicones entrant dans cette classe sont les silicones commercialisées par la société GOLDSCHMIDT sous les dénominations ABIL QUAT 3270, ABIL QUAT 3272, ABIL QUAT 3474.
e) les silicones aminées de formule (XIII) : 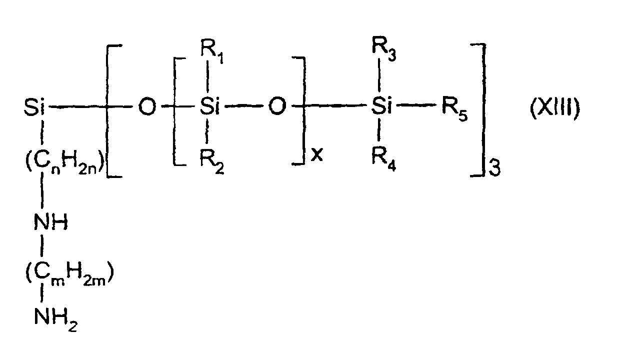 dans laquelle:
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Selon l'invention, les silicones aminées peuvent se présenter sous formes d'huile, de solutions aqueuses, alcooliques ou hydroalcooliques, sous forme de dispersion ou d'émulsion.
Une forme de réalisation particulièrement intéressante est leur utilisation sous forme d'émulsions en particulier sous forme de microémulsions ou de nanoémulsions.

On peut utiliser par exemple le produit commercialisé sous la dénomination "Emulsion Cationique DC 929" par la Société Dow Corning qui comprend, outre l'amodiméthicone, un agent de surface cationique dérivés des acides gras du suif dénomméTallowtrimonium(CTFA), en association avec un agent de surface non ionique connu sous la dénomination "Nonoxynol 10".

On peut également utiliser par exemple le produit commercialisé sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit commercialisé sous la dénomination "Dow Corning Q2 7224" par la Société Dow Corning comportant en association le triméthylsilylamodiméthicone de formule (X), un agent de surface non ionique de formule : C₈H₁₇-C₆H₄-OCH₂CH₂)ₙ-OH où n = 40 dénommé encore octoxynol-40, un autre agent de surface non ionique de formule : C₁₂H₂₅-(OCH₂-CH₂)ₙ-OH où n = 6 encore dénommé isolaureth-6, et du glycol.

Les tensioactifs cationiques de type sels d'ammonium quaternaires selon l'invention sont généralement choisis parmi :
A) les sels d'ammonium quaternaires de la formule générale (XIV) suivante : 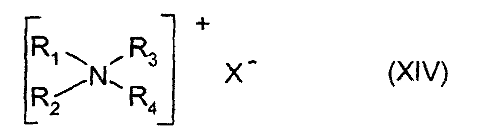 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   i) les radicaux R₁ à R₃, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R₄ désigne un radical alkyle, linéaire ou ramifié, comportant de 16 à 30 atomes de carbone.
      De préférence le tensioactif cationique est un sel (par exemple chlorure) de béhényl triméthyl ammonium.
   ii) les radicaux R₁ et R₂, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R₃ et R₄, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XV) suivante : 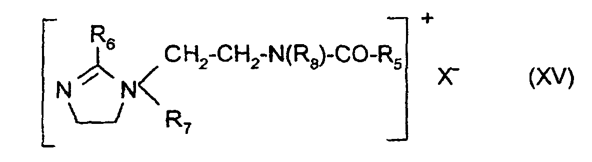 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XVI) : 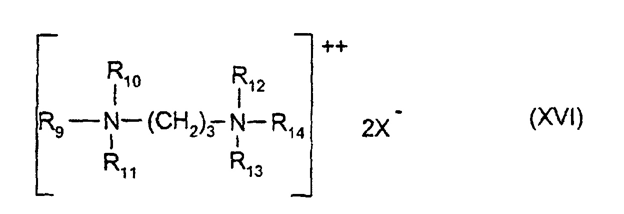 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XVII) suivante : 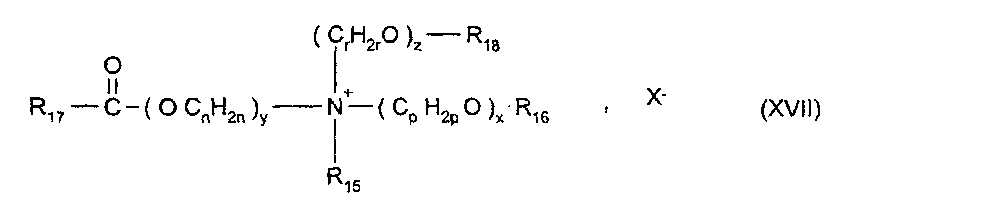 dans laquelle :
   - R₁₅ est choisi parmi les radicaux alkyles en C₁-C₆ et les radicaux hydroxyalkyles ou dihydroxyalkyles en C₁-C₆;
   - R₁₆ est choisi parmi :
      - le radical R₁₉ 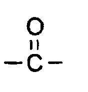
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 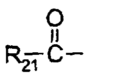
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XVII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 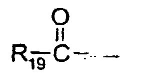
   - les radicaux méthyle, éthyle ou hydrocarbonés en C₁₄-C₂₂
   - l'atome d'hydrogène ;

- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi:
   - le radical 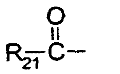
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Les cyclopolymères d'alkyl diallyl amine ou de dialkyl diallyl ammonium sont choisis parmi les homopolymères ou copolymères comportant comme constituant principal de la chaîne des motifs répondant aux formules (XVIII) ou (XIX) : 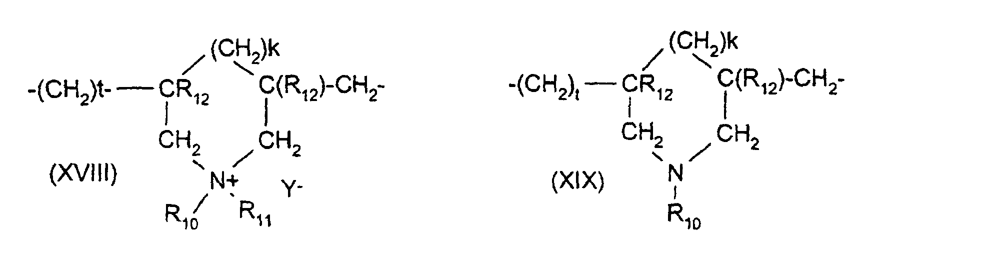 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".

Il est bien entendu possible de mettre en oeuvre des mélanges d'agents conditionneurs.

Selon l'invention, le ou les agents conditionneurs cationiques peuvent représenter de 0,001 % à 10 % en poids, de préférence de 0,01 % à 5% en poids et plus particulièrement de 0,1 à 3% en poids par rapport au poids total de la composition finale.

Les compositions de l'invention peuvent en outre contenir avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Coco-amphodipropionic acid.
A titre d'exemple on peut citer le cocoamphodiacetate commercialisé sous la dénomination commerciale MIRANOL C2M concentré par la société RHONE POULENC.

Dans les compositions conformes à invention, on peut utiliser des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise alors de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La composition de l'invention peut également contenir au moins un additif choisi parmi les épaississants (associatif ou non associatif), les parfums, les agents nacrants, les conservateurs, les filtres solaires siliconés ou non, les vitamines, les provitamines, les polymères cationiques autres que ceux de l'invention, amphotères, anioniques ou non ioniques, les protéines, les hydrolysats de protéïne, l'acide méthyl-18 eicosanoique, les hydroxyacides, le panthénol, les silicones volatiles ou non volatiles, cycliques ou linéaires ou réticulés, modifiées ou non, et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions selon l'invention présentent un pH final généralement compris entre 2 et 10. De préférence, ce pH est compris entre 3 et 6,5. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon invention peuvent être des compositions d'après-shampooing à rincer ou non.
Les compositions selon l'invention peuvent être des compositions détergentes telles que des shampooings, des gels-douche, des bains moussants et peuvent être également des démaquillants. Dans ce mode de réalisation de l'invention, les compositions comprennent une base lavante, généralement aqueuse.

Le ou les tensioactifs formant éventuellement la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants. Dans les exemples, MA signifie matière active.

Dans les exemples, les noms commerciaux ont les définitions suivantes :

### EXEMPLE 1

On a réalisé un après-shampooing à rincer conforme à l'invention de composition suivante :

| Composition | Invention A | B |
|---|---|---|
| Fécule de pomme de terre modifiée par l'acide 2-chloroéthyl aminodipropionique neutralisée par la soude, (Structure Solanace de NATIONAL STARCH) | 1.5 g | 1.5 g |
| Homopolymère de chlorure de diallyl diméthyl ammonium en solution aqueuse à 40% MA (MERQUAT 100 de CALGON) | 0.5 g MA | |
| Polymère amphotère: Terpolymère de chlorure de diallyl diméthyl ammonium, d'acide acrylique et d'acrylamide en solution aqueuse à 40% MA (MERQUAT 3300 de CALGON) | - | 0.5 g MA |
| Eau déminéralisée q.s.p. | 100.0 g | 100.0 g |

On applique ces compositions sur des cheveux lavés et essorés. On laisse pauser pendant 2 minutes, puis on rince à l'eau.
Les cheveux traités avec la composition A selon invention sont plus lisses et plus souples à l'état mouillé et plus gonflant et plus légers à l'état séché que les cheveux traités avec la composition B.

### EXEMPLE 2

On a réalisé un après-shampooing à rincer conforme à l'invention de composition suivante :
- Fécule de pomme de terre modifiée par l'acide 2-chloroéthyl aminodipropionique neutralisée par la soude (Structure Solanace de NATIONAL STARCH) 1,5 g
- Mélange de myristate, palmitate et stéarate de myristyle, cétyle et stéaryle 0,5 g
- Amodiméthicone vendue en émulsion cationique à 35% de matière active (FLUID DC 939 de DOW CORNING) 1,4 g MA
- Chlorure de béhényl triméthyl ammonium en solution aqueuse à 80% de MA (GENAMIN KDMP de CLARIANT) 1,2 g MA
- Mélange d'alcool cétylique et d'alcool stéarylique (50/50 en poids) 2,5 g
- Lauryldiméthiconecopolyol à 91% de MA (Q2-5200 de DOW CORNING) 0,23 gMA
- Acide citrique 0,1 g
- Parfum, conservateurs qs
- Eau qsp 100g

Les cheveux traités avec la composition selon l'invention sont lisses et souples à l'état mouillé et gonflant et légers à l'état séché.

### EXEMPLE 3

On a réalisé un shampooing conforme à l'invention de composition suivante :

| | |
|---|---|
| Lauryléthersulfate de sodium (C12/C14 à 70/30) à 2.2 moles d'oxyde d'éthylène | 15,5 g MA |
| Cocoyl bétaïne en solution aqueuse à 32% M.A. | 3 g MA |
| Chlorure d'hydroxypropyl guar triméthyl ammonium, vendu sous la dénomination Jaguar C13S par la société RHODIA | 0.1 g |
| Fécule de pomme de terre modifiée par l'acide 2-chloroéthyl aminodipropionique neutralisée par la soude (Structure Solanace de NATIONAL STARCH) | 0.3 g |
| Polydiméthylsiloxane de viscosité 60 000 cSt | 2.7 g |
| Amodiméthicone en émulsion cationique à 35% de MA (DC939 de DOW CORNING) | 1,05 gMA |
| Mélange 1-(hexadécyloxy) 2-octadécanol / Alcool cétylique | 2,5 g |
| Monoisopropanolamide de coprah | 0.5 g |
| Conservateurs, parfum | q.s. |
| Acide citrique q.s. | pH 5,5 |
| Eau déminéralisée q.s.p. | 100 g |

On effectue un shampooing en appliquant environ 12 g de la composition sur des cheveux préalablement mouillés. On fait mousser le shampooing puis on rince abondamment à l'eau.
Les cheveux traités avec cette composition sont doux, légers et se démêlent facilement.

### EXEMPLE 4

On a réalisé un après-shampooing à rincer conforme à l'invention de composition suivante :
- Fécule de pomme de terre modifiée par l'acide 2-chloroéthyl aminodipropionique neutralisée par la soude (Structure Solanace de NATIONAL STARCH) 1 g
- Mélange de myristate, palmitate et stéarate de myristyle, cétyle et stéaryle 0,5 g
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 1,4 g MA
- Chlorure de béhényl triméthyl ammonium en solution aqueuse à 80% de MA (GENAMIN KDMP de CLARIANT) 1,2 g MA
- Mélange d'alcool cétylique et d'alcool stéarylique (50/50 en poids) 3 g
- Lauryldiméthiconecopolyol à 91% de MA (Q2-5200 de DOW CORNING) 0,23 gMA
- Acide citrique 0,1 g
- Parfum, conservateurs qs
- Eau qsp 100 g

### EXEMPLE 5

On a réalisé un après-shampooing à rincer conforme à invention de composition suivante :
- Fécule de pomme de terre modifiée par l'acide 2-chloroéthyl aminodipropionique neutralisée par la soude (Structure Solanace de NATIONAL STARCH) 1 g
- Cire de candelilla 0,3 g
- N-oléoyl dihydrosphingosine 0,1 g
- Triméthylsilyl amodiméthicone en émulsion non ionique à 20% MA 0,92 g MA
- Chlorure de béhényl triméthyl ammonium en solution aqueuse à 80% de MA (GENAMIN KDMP de CLARIANT) 0,88 g MA
- Quaternium-87 à 75% MA dans le propylèneglycol (REWOQUAT PG 75 de REWO) 2,5 gMA
- Alcool stéarylique 1 g
- Monolaurate de sorbitane oxyéthyléné 0,3 g
- Hydrolysat de protéine de blé quatemisé 0,06 g
- Parfum, conservateurs qs
- Eau qsp 100 g

## Revendications

1. Composition cosmétique, **caractérisée par le fait qu'**elle comprend, dans un milieu cosmétiquement acceptable,
a) au moins un amidon amphotère choisi parmi les composés de formules (I) à (IV): 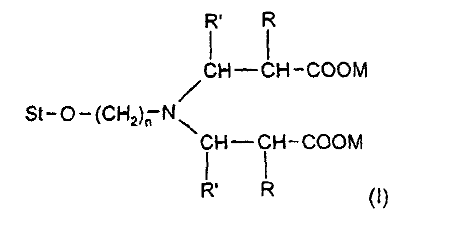 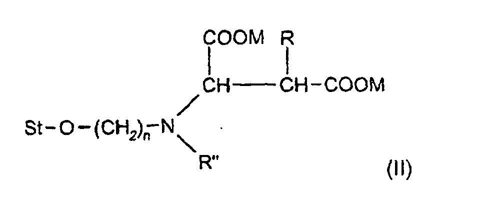 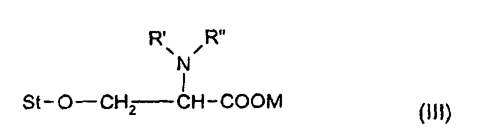 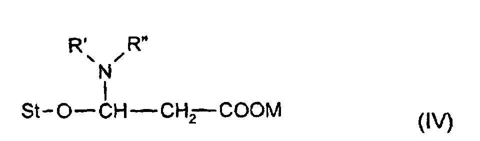 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux, NH₄, un ammonium quaternaire ou une amine organique,
R" représente un atome d'hydrogène ou un radical alkyle ayant de 1 à 18 atomes de carbone,
et b) au moins un agent conditionneur cationique choisi parmi :
- les silicones cationiques,
- les tensioactifs de type sels d'ammonium quaternaires,
- les cyclopolymères d'alkyl diallyl amine ou de dialkyl diallyl ammonium et
- les polymères de polyammonium quaternaires choisis parmi :
(1) les polymères de diammonium quaternaires contenant des motifs récurrents répondant à la formule (IV): 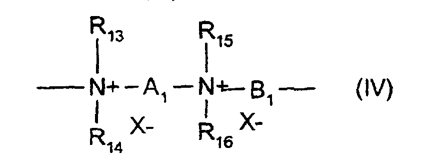 formule (IV) dans laquelle:
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH-;
(2) les polymères de polyammonium quaternaires constitués de motifs de formule (VII): 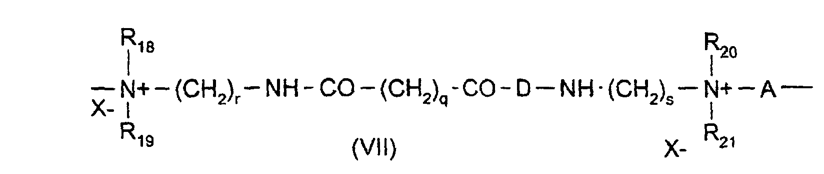 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X⁻ désigne un anion d'un acide minéral ou organique tel qu'un halogènure,
D peut être nul ou peut représenter un groupement -(CH₂)ₜ-CO- dans lequel t désigne un nombre égal à 4 ou à 7,
A désigne un radical d'un dihalogénure ou représente de préférence -CH₂-CH₂-O-CH₂-CH₂-.

2. Composition selon la revendication précédente, **caractérisée par le fait que** les amidons sont de formules (I) ou (II).

3. Composition selon la revendication précédente, **caractérisée par le fait que** R, R', R" représentent un atome d'hydrogène et n est égal à 2.

4. Composition selon l'une quelconque des revendications précédentes, **caractérisée par le fait que** la silicone aminée est choisie parmi :
(a) les polysiloxanes dénommés dans le dictionnaire CTFA "amodiméthicone" et répondant à la formule : 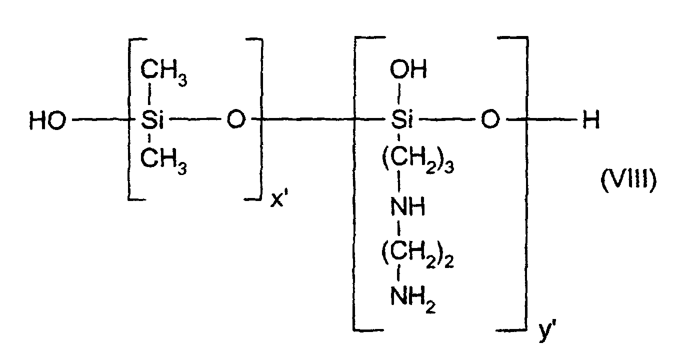 dans laquelle x' et y' sont des nombres entiers dépendant du poids moléculaire, généralement tels que ledit poids moléculaire moyen en nombre est compris entre 5 000 et 500 000 ;
(b) les silicones aminées répondant à la formule :
R'ₐG₃₋ₐ-Si(OSiG₂)ₙ-(OSiG_{b}R'_{2-b})ₘ-O-4-SiG₃₋ₐ-R'ₐ (IX)
dans laquelle:
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000
et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quaternisé choisi parmi les groupements :
-NR"-CH₂-CH₂-N'(R")₂
-N(R")₂
-N^{⊕}(R")₃ A⁻
-NH^{⊕}(R")₂ A⁻
-NH₂^{⊕}(R") A⁻
-N(R")-CH₂-CH₂-N^{⊕}R" H₂ A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
(c) les silicones aminées répondant à la formule : 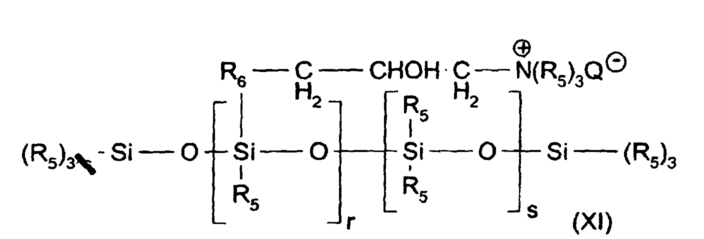 dans laquelle:
R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
Q⁻ est un anion tel qu'un ion halogénure, notamment chlorure ou un sel d'acide organique
r représente une valeur statistique moyenne de 2 à 20 et en particulier de 2 à 8 ;
s représente une valeur statistique moyenne de 20 à 200 et en particulier de 20 à 50.
d) les silicones ammonium quaternaire de formule : 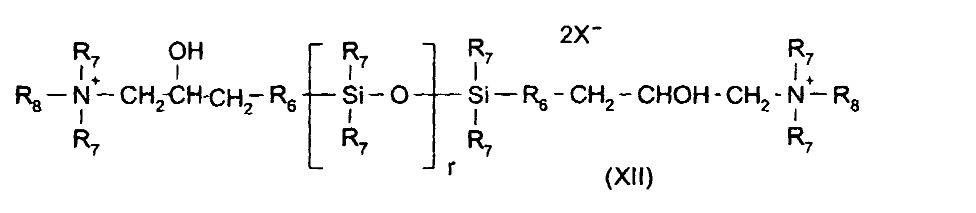 dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;
e) les silicones aminées de formule (XIII) : 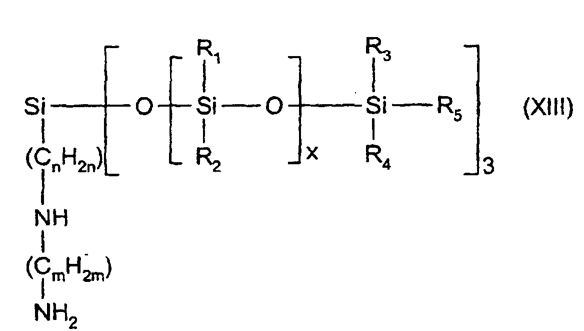 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

5. Composition selon l'une quelconque des revendications 1 à 3, **caractérisée en ce que** les tensioactifs de type sels d'ammonium quaternaires sont choisis parmi :
A) les sels d'ammonium quaternaires de la formule générale (XIV) suivante : 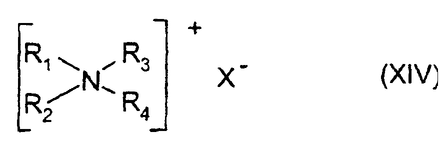 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
et
i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes,
R4 désigne un radical alkyle, linéaire ou ramifié, comportant de 16 à 30 atomes de carbone.
ii) les radicaux R₁ et R₂, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
R₃ et R₄, identiques ou différents, désignent un radical alkyle, linéaire ou ramifié, comportant de 12 à 30 atomes de carbone, ledit radical comprenant au moins une fonction ester ou amide.
R₃ et R₄ sont notamment choisis parmi les radicaux alkyl(C₁₂-C₂₂)amido alkyle(C₂-C₆), alkyl(C₁₂-C₂₂)acétate ;
B) - les sels d'ammonium quaternaire de l'imidazolinium, comme par exemple celui de formule (XV) suivante : 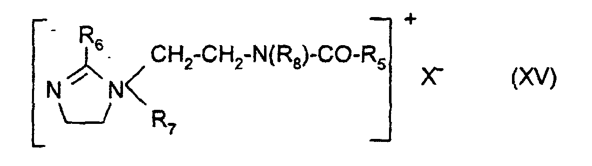 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates.
C) - les sels de diammonium quaternaire de formule (XVI) : 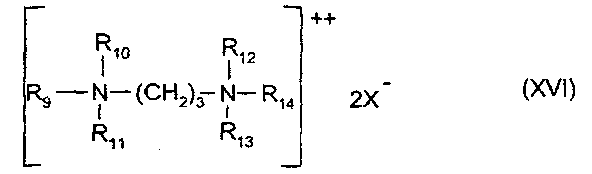 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X- est un anion choisi dans le groupe des halogénures, acétates, phosphates, nitrates et méthylsulfates.
D) - les sels d'ammonium quaternaire contenant au moins une fonction ester de formule (XVII) suivante : 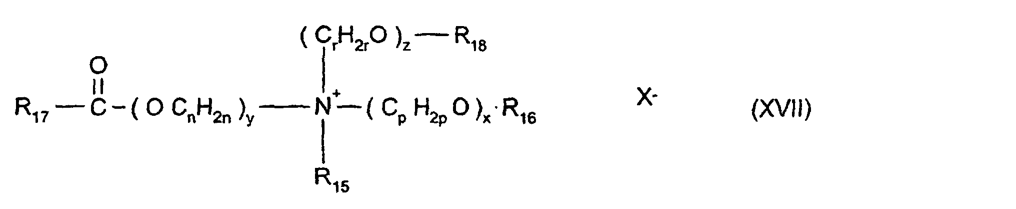
dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
- le radical 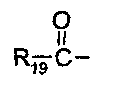
- les radicaux R₂₀ hydrocarbonés en C₁-C₂₂ linéaires ou ramifiés, saturés ou insaturés,
- l'atome d'hydrogène,
- R₁₈ est choisi parmi :
- le radical 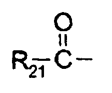
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

6. Composition selon l'une quelconque des revendications précédentes, **caractérisée par le fait que** ledit tensioactif cationique est choisi parmi les sels de béhényl triméthyl ammonium, les sels de stéaramidopropyl diméthyl (myristylacétate) ammonium, le Quaternium-27 ou le Quaternium-83.

7. Composition selon l'une quelconque des revendications précédentes **caractérisée en ce que** les cyclopolymères d'alkyl diallyl amine ou de dialkyl diallyl ammonium sont choisis parmi les homopolymères ou copolymères comportant comme constituant principal de la chaîne des motifs répondant aux formules (XVIII) ou (XIX) : 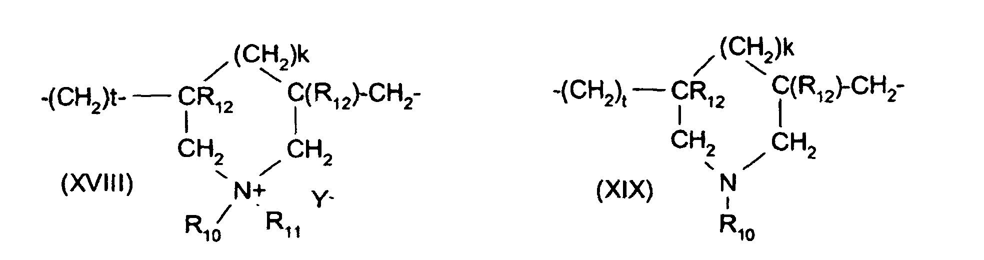 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.

8. Composition selon l'une quelconque des revendications 1 à 5, **caractérisée en ce que** les polymères de diammonium quaternaires contenant des motifs récurrents répondant à la formule (VI) sont constitués de motifs récurrents répondant à la formule : 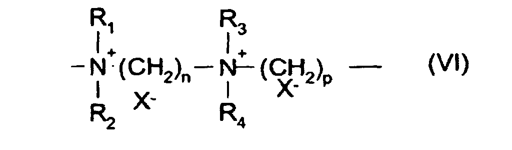 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

9. Composition selon l'une quelconque des revendications 1 à 8, **caractérisée par le fait que** l'amidon amphotère est présent dans des concentrations comprises entre 0,01 et 10 %, et de préférence entre 0,1 et 5 % en poids par rapport au poids total de la composition.

10. Composition selon l'une quelconque des revendications précédentes, **caractérisée en ce que** l'agent conditionneur cationique est présent à une concentration comprise entre 0,001 % et 10 % en poids par rapport au poids total de la composition, de préférence entre 0,01 % et 5 % en poids.

11. Composition selon l'une quelconque des revendications précédentes, **caractérisée par le fait qu'**elle comprend en outre au moins un agent tensioactif choisi parmi les tensioactifs anioniques, non ioniques, amphotères et leurs mélanges.

12. Composition selon la revendication 11, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

13. Composition selon l'une quelconque des revendications 1 à 12, **caractérisée par le fait qu'**elle comprend au moins un additif choisi parmi les épaississants, les parfums, les agents nacrants, les conservateurs, les filtres solaires siliconés ou non, les vitamines, les provitamines, les polymères cationiques, amphotères, anioniques ou non ioniques, les protéines, les hydrolysats de protéïne, l'acide méthyl-18 eicosanoique, les hydroxyacides, le panthénol, les silicones volatiles ou non volatiles, cycliques ou linéaires ou réticulés, modifiées ou non.

14. Composition selon l'une quelconque des revendications 1 à 13, **caractérisée par le fait qu'**elle présente un pH compris entre 2 et 10.

15. Composition selon l'une quelconque des revendications 1 à 14, **caractérisée par le fait qu'**elle présente un pH compris entre 3 et 6,5.

16. Composition selon l'une quelconque des revendications précédentes, **caractérisée par le fait qu'**elle se présente sous forme de shampooing, d'après-shampooing à rincer ou non, de composition pour la permanente, le défrisage, la coloration ou la décoloration des cheveux, de composition à rincer à appliquer entre les deux étapes d'une permanente ou d'un défrisage, de gels-douche, de bains moussants et de démaquillants.

17. Utilisation d'une composition telle que définie dans l'une quelconque des revendications précédentes pour le lavage ou pour le soin des matières kératiniques.

18. Procédé de traitement des matières kératiniques, telles que les cheveux, **caractérisé en ce qu'**il consiste à appliquer sur lesdites matières une composition cosmétique selon l'une des revendications 1 à 16, puis à effectuer éventuellement un rinçage à l'eau.

## Patentansprüche

1. Kosmetische Zusammensetzung, **dadurch gekennzeichnet, dass** sie in einem kosmetisch akzeptablen Medium enthält:
(a) mindestens eine amphotere Stärke, die unter den Verbindungen der Formeln (I) bis (IV) ausgewählt ist: 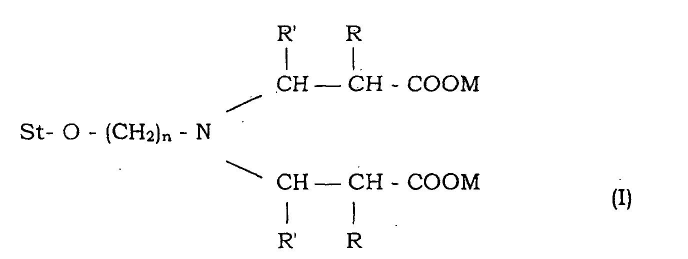 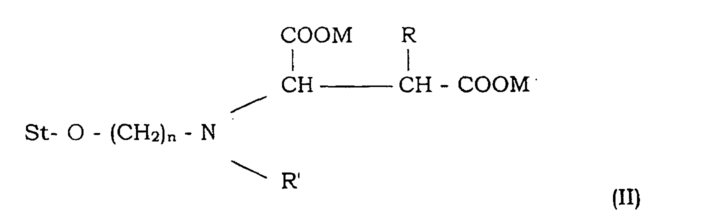 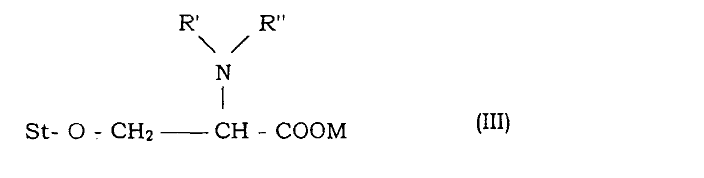 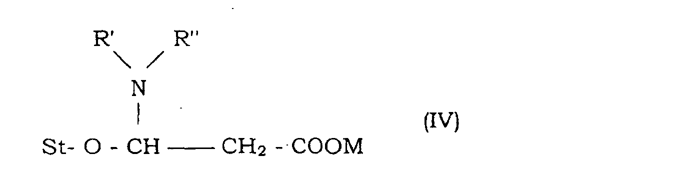 wobei in den Formeln bedeuten:
· St- O ein Stärkemolekül,
· die Gruppen R, die gleich oder verschieden sind, ein Wasserstoffatom oder die Methylgruppe,
· die Gruppen R', die gleich oder verschieden sind, ein Wasserstoffatom, die Methylgruppe oder-COOH,
· n 2 oder 3,
· die Gruppen M, die gleich oder verschieden sind, ein Wasserstoffatom, ein Alkali- oder Erdalkalimetall, NH₄⁺, eine quartäre Ammoniumgruppe oder ein organisches Amin,
· R" ein Wasserstoffatom oder eine Alkylgruppe mit 1 bis 18 Kohlenstoffatomen,
(b) mindestens ein kationisches Konditioniermittel, das ausgewählt ist unter:
· kationischen Siliconen,
· grenzflächenaktiven Stoffen von Typ der quartären Ammoniumsalze,
· Alkyldiallylamin- oder Dialkyldiallylammonium-Cyclopolymeren, und
· quartären Polyammoniumverbindungen, die ausgewählt sind unter:
(1) Quartären Diammoniumpolymeren mit wiederkehrenden Einheiten der folgenden Formel (IV): 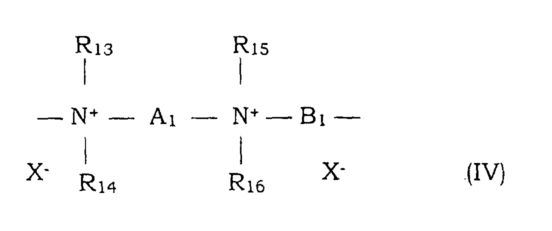 wobei in der Formel (IV) die Gruppen R₁₃, R₁₄, R₁₅ und R₁₆, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder wobei die Gruppen R₁₃, R₁₄, R₁₅ und R₁₆ gemeinsam oder unabhängig voneinander mit den Stickstoffatomen, an die sie gebunden sind, Heterocyclen bilden, die gegebenenfalls ein zweites Heteroatom enthalten, das von Stickstoff verschieden ist, oder wobei die Gruppen R₁₃, R₁₄, R₁₅ und R₁₆ eine geradkettige oder verzweigte C₁₋₆-Alkylgruppe bedeuten, die mit einer Nitril-, Ester-, Acyl- oder Amidgruppe oder -CO-O-R₁₇-D oder -CO-NH-R₁₇-D substituiert ist, worin R₁₇ eine Alkylengruppe und D eine quartäre Ammoniumgruppe bedeuten;
A₁ und B₁ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
X⁻ bedeutet ein Anion, das von einer anorganischen oder organischen Säure abgeleitet ist;
die Gruppen A₁, R₁₃ und R₁₅ können mit den beiden Stickstoffatomen, an die sie gebunden sind, einen Piperazinring bilden; wenn A₁ eine geradkettige oder verzweigte, gesättigte oder ungesättigte Alkylengruppe oder Hydroxyalkylengruppe bedeutet, kann die Gruppe B₁ auch eine Gruppe (CH₂)ₙ-CO-D-OC-(CH₂)ₙ-bedeuten, worin D bedeutet:
a) eine Glykolgruppe der Formel: -O-Z-O- , worin Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃)-O]_{y}-CH₂-CH(CH₃)-
worin x und y ganze Zahlen von 1 bis 4 bedeuten und einen wohl definierten und einzigen Polymerisationsgrad darstellen, oder beliebige Zahlen von 1 bis 4 bedeuten und einen mittleren Polymerisationsgrad darstellen;
b) ein bis-sekundäres Diamin, beispielsweise ein Piperazinderivat,
c) ein bis-primäres Diamin der Formel : -NH-Y-NH- ,
worin Y eine geradkettige oder verzweigte Kohlenwasserstoffgruppe oder auch die zweiwertige Gruppe -CH₂-CH₂-S-S-CH₂-CH₂- bedeutet, oder
d) die Ureylengruppe der Formel : -NH-CO-NH-;
(2) Quartäre Polyammonium-Polymere, die aus Einheiten der folgenden Formel (VII) bestehen: 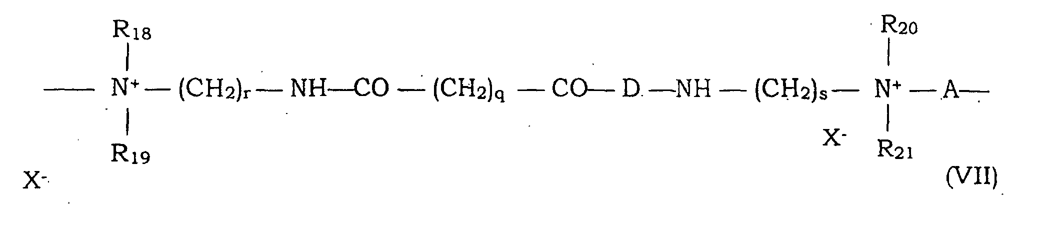
wobei in der Formel bedeuten:
R₁₈, R₁₉, R₂₀ und R₂₁, die identisch oder voneinander verschieden sind, ein Wasserstoffatom oder Methyl, Ethyl,
Propyl, β-Hydroxyethyl, β-Hydroxypropyl oder-CH₂CH₂(OCH₂CH₂)ₚOH, wobei p Null oder eine ganze Zahl im Bereich von 1 bis 6 bedeutet, mit der Maßgabe, dass R₁₈, R₁₉, R₂₀ und R₂₁ nicht gleichzeitig Wasserstoff bedeuten,
r und s, die gleich oder verschieden sind, eine ganze Zahl im Bereich von 1 bis 6,
q Null oder eine ganze Zahl im Bereich von 1 bis 34,
X⁻ ein Anion einer anorganischen oder organischen Säure, beispielsweise ein Halogenid,
D kann nicht vorhanden sein oder -(CH₂)ₜ-CO- bedeuten, wobei t 4 oder 7 ist, und
A ein Anion, z.B. ein Dihalogenid, oder vorzugsweise -CH₂-CH₂-O-CH₂-CH₂-

2. Zusammensetzung nach dem vorhergehenden Anspruch, **dadurch gekennzeichnet, dass** die Stärkeverbindung ein Verbindung der Formel (I) oder (II) ist.

3. Zusammensetzung nach dem vorhergehenden Anspruch, **dadurch gekennzeichnet, dass** die Gruppen R , R' und R" ein Wasserstoffatom bedeuten und n 2 ist.

4. Zusammensetzung nach einem der vorhergehenden Ansprüche, **dadurch gekennzeichnet, dass** das aminierte Silicon ausgewählt ist unter:
a) Polysiloxanen, die nach CTFA-Nomenklatur als "Amodimethicon" bezeichnet werden und der folgenden Formel entsprechen: 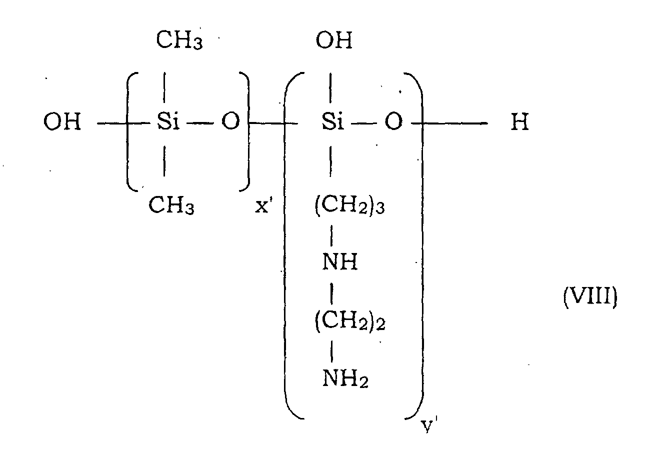 worin x' und y' ganze Zahlen bedeuten, die von der Molmasse abhängen und im Allgemeinen so gewählt sind, dass die zahlenmittlere Molmasse im Bereich von 5 000 bis 500 000 liegt;
b) aminierten Siliconen der folgenden Formel:
R'ₐG₃₋ₐ-Si(OSiG₂)ₙ-(OSiG_{b}R'_{2-b})ₘ-O-SiG₃₋ₐ-R'ₐ (IX),
worin bedeuten:
· die Gruppe G Wasserstoff, Phenyl, OH oder C₁₋₈-Alkyl, beispielsweise Methyl,
· a 0 oder eine ganze Zahl von 1 bis 3 und insbesondere 0,
· b 0 oder 1 und insbesondere 1,
· mund n Zahlen, die so ausgewählt sind, dass die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
· die Gruppe R' eine einwertige Gruppe der Formel -C_{q}H_{2q}L,
worin q eine Zahl von 2 bis 8 ist und L eine gegebenenfalls quaternisierte, aminierte Gruppe bedeutet, welche unter den folgenden Gruppen ausgewählt ist:
-N(R")-CH₂-CH₂-N(R")₂
-N(R")₂
-N^{⊕}(R")₃A⁻
-NH^{⊕}(R")₂A⁻
-NH₂^{⊕}(R") A⁻
-N(R")-CH₂-CH₂-NH₂^{⊕}(R") A⁻,
worin die Gruppen R" Wasserstoff, Phenyl, Benzyl oder eine einwertige gesättigte Kohlenwasserstoffgruppe, beispielsweise eine Alkylgruppe mit 1 bis 20 Kohlenstoffatomen, bedeuten können und A- ein Halogenid ist, wie beispielsweise Fluorid, Chlorid, Bromid oder Iodid;
c) aminierten Siliconen der folgenden Formel: 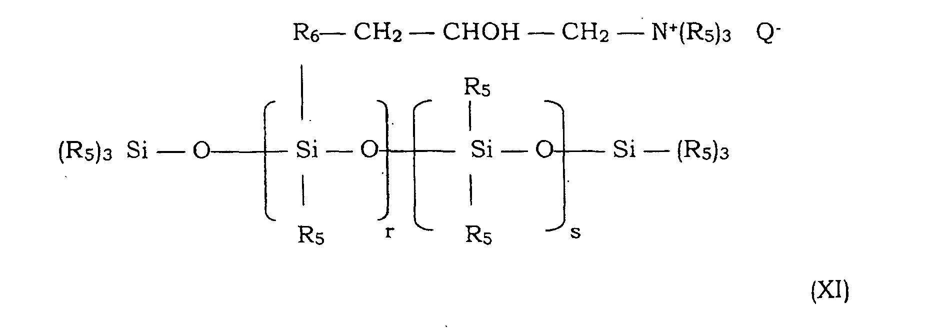 worin bedeuten:
- R₅ eine einwertige Kohlenwasserstoffgruppe mit 1 bis 18 Kohlenstoffatomen und insbesondere eine C₁₋₁₈-Alkylgruppe oder eine C₂₋₁₈-Alkenylgruppe, beispielsweise Methyl,
- R₆ eine zweiwertige Kohlenwasserstoffgruppe und insbesondere eine Alkylengruppe mit 1 bis 18 Kohlenstoffatomen oder eine zweiwertige Alkylenoxygrüppe mit 1 bis 18 Kohlenstoffatomen, beispielsweise mit 1 bis 8 Kohlenstoffatomen, die über eine Si-C-Bindung an Si gebunden ist,
- Q⁻ ein Anion, z.B. Halogenid, insbesondere Chlorid, oder ein Salz einer organischen Säure,
- r einen statistischen Mittelwert von 2 bis 20 und insbesondere von 2 bis 8, und
- s einen statistischen Mittelwert von 20 bis 200 und insbesondere von 20 bis 50;
d) quartären Ammoniumsiliconen der folgenden Formel: 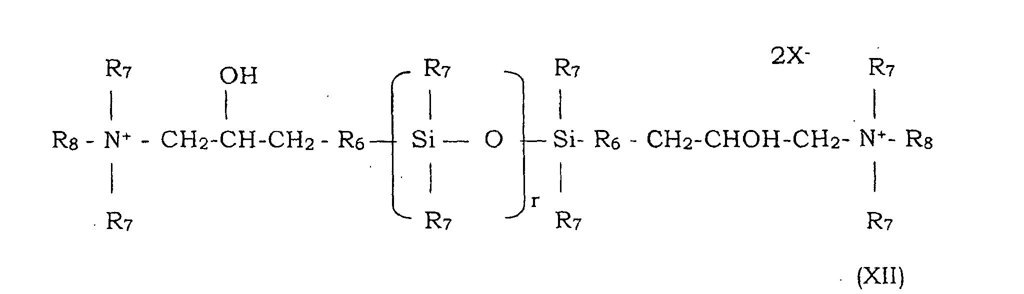 worin bedeuten:
- die Gruppen R₇, die gleich oder verschieden sind, eine einwertige Kohlenwasserstoffgruppe mit 1 bis 18 Kohlenstoffatomen und insbesondere eine C₁₋₁₈-Alkylgruppe, eine C₂₋₁₈-Alkenylgruppe oder einen Ring mit 5 oder 6 Kohlenstoffatomen, beispielsweise Methyl,
- R₆ eine zweiwertige Kohlenwasserstoffgruppe und insbesondere eine Alkylengruppe mit 1 bis 18 Kohlenstoffatomen oder eine zweiwertige Alkylenoxygruppe mit 1 bis 18 Kohlenstoffatomen, beispielsweise mit 1 bis 8 Kohlenstoffatomen, die über eine SiC-Bindung an Si gebunden ist,
- die Gruppen R₈, die gleich oder verschieden sind, Wasserstoff, eine einwertige Kohlenwasserstoffgruppe mit 1 bis 18 Kohlenstoffatomen und insbesondere eine C₁₋₁₈-Alkylgruppe, eine C₂₋₁₈-Alkenylgruppe oder -R₆-NHCOR₇,
- X⁻ ein Anion, beispielsweise ein Halogenid, insbesondere Chlorid, oder ein Salz einer organischen Säure (Acetat...),
- r einen statistischen Mittelwert von 2 bis 200 und insbesondere von 5 bis 100;
e) aminierten Siliconen der Formel (XIII): 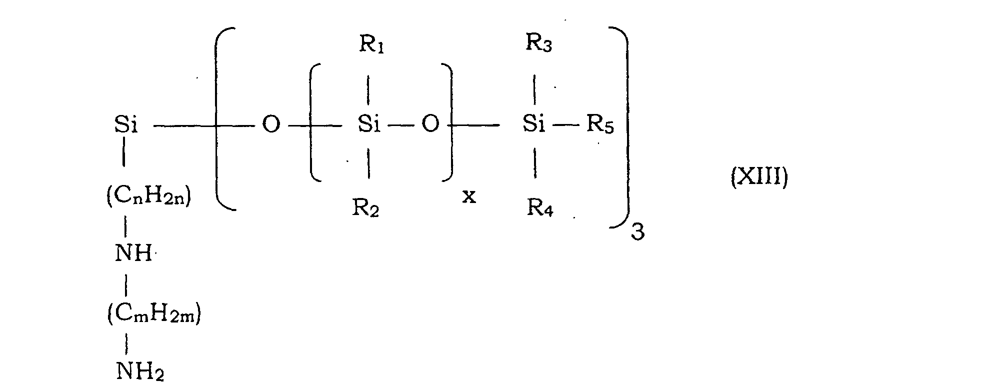 worin bedeuten:
- die Gruppen R₁, R₂, R₃ und R₄, die gleich oder verschieden sind, C₁₋₄-Alkyl oder Phenyl,
- R₅ C₁₋₄-Alkyl oder Hydroxy,
- n eine ganze Zahl von 1 bis 5,
- m eine ganze Zahl von 1 bis 5,
- wobei x so ausgewählt ist, dass der Aminoindex im Bereich von 0,01 bis 1 meq liegt,

5. Zusammensetzung nach einem der Ansprüche 1 bis 3, **dadurch gekennzeichnet, dass** die grenzflächenaktiven Stoffe vom Typ der quartären Ammoniumsalze ausgewählt sind unter:
A) den quartären Ammoniumsalzen der folgenden allgemeinen Formel (XIV): 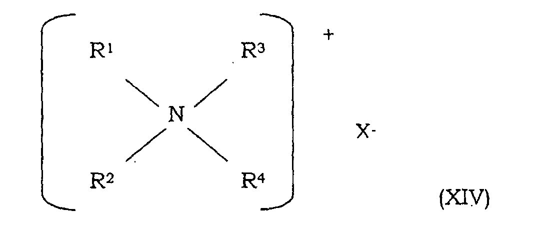 worin X ein Anion bedeutet, das unter den Halogeniden (Chlorid, Bromid oder Iodid) oder C₂₋₆-Alkylsulfaten und insbesondere Methylsulfat, Phosphaten, Alkyl- oder Alkylarylsulfonaten und von organischen Säuren abgeleiteten Anionen, wie Acetat oder Lactat, ausgewählt ist; und
i) die Gruppen R₁ bis R₃, die gleich oder verschieden sein können, bedeuten eine geradkettige oder verzweigte aliphatische Gruppe mit 1 bis 4 Kohlenstoffatomen oder eine aromatische Gruppe, wie Aryl oder Alkylaryl. Die aliphatischen Gruppen können Heteroatome enthalten, wie beispielsweise insbesondere Sauerstoff, Stickstoff, Schwefel und Halogene;
R₄ bedeutet eine geradkettige oder verzweigte Alkylgruppe mit 16 bis 30 Kohlenstoffatomen;
ii) die Gruppen R₁ und R₂, die gleich oder verschieden sein können, bedeuten eine geradkettige oder verzweigte aliphatische Gruppe mit 1 bis 4 Kohlenstoffatomen oder eine aromatische Gruppe, wie Aryl oder Alkylaryl. Die aliphatischen Gruppen können Heteroatome enthalten, wie insbesondere Sauerstoff, Stickstoff, Schwefel und Halogene; die aliphatischen Gruppen sind beispielsweise unter den Gruppen Alkyl, Alkoxy, Alkylamid und Hydroxyalkyl ausgewählt, die etwa 1 bis 4 Kohlenstoffatome enthalten; die Gruppen R₃ und R₄, die gleich oder verschieden sind, bedeuten eine geradkettige oder verzweigte Alkylgruppe mit 12 bis 30 Kohlenstoffatomen, wobei die Gruppe mindestens eine Ester- oder Amidfunktion enthält; die Gruppen R₃ und R₄ sind insbesondere unter den Gruppen Alkyl(C₁₂₋₂₂)amido-C₂₋₆-alkyl oder Alkyl(C₁₂-₂₂)acetat ausgewählt.
B) den quartären Imidazoliniumsalzen, beispielsweise den Salzen der folgenden Formel (XV): 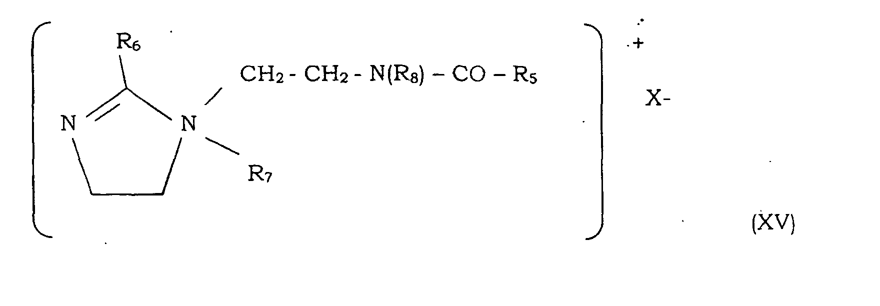 worin Rs eine Alkenyl- oder Alkylgruppe mit 8 bis 30 Kohlenstoffatomen bedeutet, die beispielsweise von Talgfettsäuren abgeleitet sind, R₆ ein Wasserstoffatom, eine C₁₋₄-Alkylgruppe oder eine Alkenyl- oder Alkylgruppe mit 8 bis 30 Kohlenstoffatomen bedeutet, R₇ eine C₁₋₄-Alkylgruppe bedeutet, R₈ ein Wasserstoffatom oder eine C₁₋₄-Alkylgruppe bedeutet und X ein Anion ist, das unter den Halogeniden, Phosphaten, Acetaten, Lactaten, Alkylsulfaten und Alkyl- oder Alkylarylsulfonaten ausgewählt ist;
C) den quartären Diammoniumsalzen der Formel (XVI): 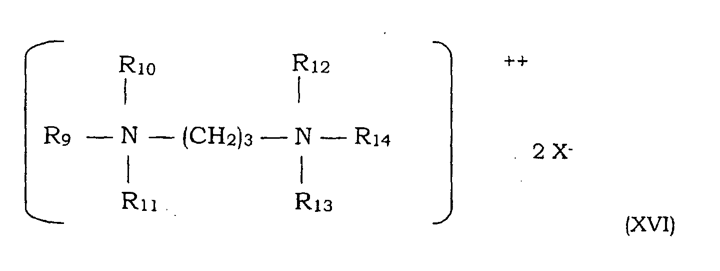 worin R₉ eine aliphatische Gruppe mit etwa 16 bis 30 Kohlenstoffatomen bedeutet, R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind und X ein Anion ist, das unter den Halogeniden, Acetaten, Phosphaten, Nitraten und Methylsulfaten ausgewählt ist;
D) quartären Ammoniumsalzen, die mindestens eine Esterfunktion enthalten, der folgenden Formel (XVII): 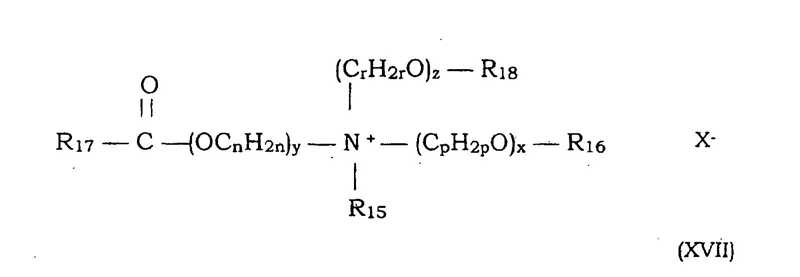 wobei in der Formel:
- R₁₅ unter den C₁₋₆-Alkylgruppen und den Hydroxyalkyloder Dihydroxyalkylgruppen mit 1 bis 6 Kohlenstoffatomen ausgewählt ist;
- R₁₆ ausgewählt ist unter:
- der Gruppe 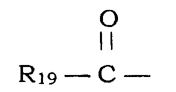
- den gesättigten oder ungesättigten, geradkettigen oder verzweigten Gruppen R₂₀ auf Kohlenwasserstoffbasis mit 1 bis 22 Kohlenstoffatomen,
- dem Wasserstoffatom,
- R₁₈ ausgewählt ist unter:
- der Gruppe 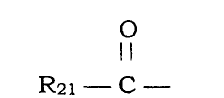
- den gesättigten oder ungesättigten, geradkettigen oder verzweigten Gruppen R₂₂ auf Kohlenwasserstoffbasis mit 1 bis 6 Kohlenstoffatomen,
- dem Wasserstoffatom,
- R₁₇, R₁₉ und R₂₁, die gleich oder verschieden sind, sind unter den geradkettigen oder verzweigten, gesättigten oder ungesättigten Kohlenwasserstoffgruppen mit 7 bis 21 Kohlenstoffatomen ausgewählt;
- n, p und r, die gleich oder verschieden sind, sind ganze Zahlen von 2 bis 6;
- y ist eine ganze Zahl von 1 bis 10;
- x und z, die gleich oder verschieden sind, bedeuten 0 oder ganze Zahlen von 1 bis 10; und
- X⁻ ist ein einfaches oder komplexes, organisches oder anorganisches Anion,
mit der Maßgabe, dass, wenn die Summe x + y + z im Bereich von 1 bis 15 liegt, R₁₆ R₂₀ bedeutet, wenn x 0 ist, und R₁₈ R₂₂ bedeutet, wenn z 0 ist.

6. Zusammensetzung nach einem der vorhergehenden Ansprüche, **dadurch gekennzeichnet, dass** der kationische grenzflächenaktive Stoff unter Behenyltrimethylammoniumsalzen, Stearamidopropyldimethyl(myristylacetat)ammoniumsalzen, Quaternium-27 und Quaternium-83 ausgewählt ist.

7. Zusammensetzung nach einem der vorhergehenden Ansprüche, **dadurch gekennzeichnet, dass** die Alkyldiallylamin- oder Dialkyldiallylammonium-Cyclopolymere unter den Homopolymeren oder Copolymeren ausgewählt sind, die als Hauptbestandteil der Kette Einheiten aufweisen, die den Formeln (XVIII) oder (XIX) entsprechen: 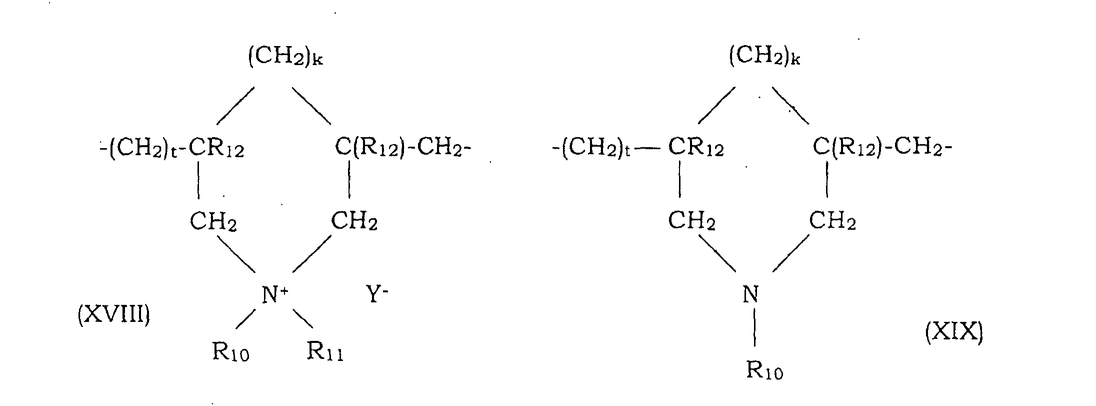 wobei in den Formeln:
k und t Null oder 1 bedeuten, wobei die Summe k + t gleich 1 ist,
R₁₂ ein Wasserstoffatom oder die Methylgruppe bedeutet, die Gruppen R₁₀ und R₁₁ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine niedere (C₁₋₄) Amidoalkylgruppe bedeuten oder R₁₀ und
R₁₁ gemeinsam mit dem Stickstoffatom, an das sie gebunden sind, heterocyclische Gruppen, wie Piperidinyl oder Morpholinyl, bilden können, und Y⁻ ein Anion, wie Bromid, Chlorid, Acetat, Borat, Citrat, Tartrat, Hydrogensulfat, Hydrogensulfit, Sulfat oder Phosphat bedeutet.

8. Zusammensetzung nach einem der Ansprüche 1 bis 5, **dadurch gekennzeichnet, dass** die quartären Diammoniumpolymere mit wiederkehrenden Einheiten der Formel (VI) aus wiederkehrenden Einheiten der folgenden Formel gebildet sind: 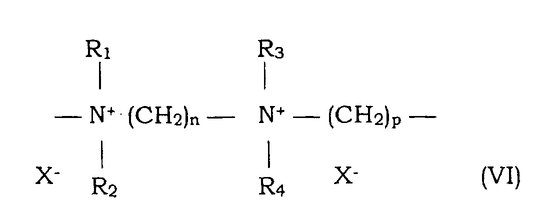 worin die Gruppen R₁, R₂, R₃ und R₄, die gleich oder verschieden sind, eine Alkyl- oder Hydroxyalkylgruppe mit etwa 1 bis 4 Kohlenstoffatomen bedeuten, n und p ganze Zahlen im Bereich von etwa 2 bis 20 bedeuten und X⁻ ein von einer anorganischen oder organischen Säure abgeleitetes Anion ist.

9. Zusammensetzung nach einem der Ansprüche 1 bis 8, **dadurch gekennzeichnet, dass** die amphotere Stärke in Konzentrationen von 0,01 bis 10 Gew.-% und vorzugsweise 0,1 bis 5 Gew.-%, bezogen auf das Gesamtgewicht der Zusammensetzung, enthalten ist.

10. Zusammensetzung nach einem der vorhergehenden Ansprüche, **dadurch gekennzeichnet, dass** das kationische Konditioniermittel in Konzentrationen von 0,001 bis 10 Gew.-%, bezogen auf das Gesamtgewicht der Zusammensetzung, und vorzugsweise 0,01 bis 5 Gew.-% enthalten ist.

11. Zusammensetzung nach einem der vorhergehenden Ansprüche, **dadurch gekennzeichnet, dass** sie ferner mindestens einen grenzflächenaktiven Stoff enthält, der unter den anionischen, nichtionischen oder amphoteren grenzflächenaktiven Stoffen oder deren Gemischen ausgewählt ist.

12. Zusammensetzung nach Anspruch 11, **dadurch gekennzeichnet, dass** der grenzflächenaktive Stoff oder die grenzflächenaktiven Stoffe in einer Menge von 0,1 bis 60 Gew.-%, vorzugsweise 3 bis 40 Gew.-% und noch bevorzugter 5 bis 30 Gew.-%, bezogen auf das Gesamtgewicht der Zusammensetzung, vorliegen.

13. Zusammensetzung nach einem der Ansprüche 1 bis 12, **dadurch gekennzeichnet, dass** sie mindestens einen Zusatzstoff enthält, der unter den Verdickungsmitteln, Parfums, Perlglanzpigmenten, Konservierungsmitteln, siliconhaltigen oder nicht siliconhaltigen Sonnenschutzfiltern, Vitaminen, Provitaminen, kationischen, amphoteren, anionischen oder nichtionischen Polymeren, Proteinen, Proteinhydrolysaten, 18-Methyleicosansäure, Hydroxysäuren, Panthenol und flüchtigen oder nicht flüchtigen, cyclischen, geradkettigen oder vernetzten, modifizierten oder nicht modifizierten Siliconen ausgewählt ist.

14. Zusammensetzung nach einem der Ansprüche 1 bis 13, **dadurch gekennzeichnet, dass** sie einen pH-Wert von 2 bis 10 aufweist.

15. Zusammensetzung nach einem der Ansprüche 1 bis 14, **dadurch gekennzeichnet, dass** sie einen pH-Wert von 3 bis 6,5 aufweist.

16. Zusammensetzung nach einem der vorhergehenden Ansprüche, **dadurch gekennzeichnet, dass** sie als Haarwaschmittel, Haarpflegemittel, das nach der Haarwäsche aufgetragen wird und ausgespült wird oder im Haar verbleibt, Zusammensetzung für eine Dauerwelle, Entkräuselung, Färbung oder Entfärbung der Haare, Zusammensetzung, die ausgespült wird und zwischen den beiden Schritten einer Dauerwelle oder Entkräuselung aufgetragen wird, Duschgel, Schaumbad oder Abschminkmittel vorliegt.

17. Verwendung einer Zusammensetzung nach einem der vorhergehenden Ansprüche zum Waschen oder zur Pflege von Keratinsubstanzen.

18. Verfahren zur Behandlung von Keratinsubstanzen, wie den Haaren, **dadurch gekennzeichnet, dass** es darin besteht, auf die Keratinsubstanzen eine kosmetische Zusammensetzung nach einem der Ansprüche 1 bis 16 aufzubringen und dann gegebenenfalls mit Wasser zu spülen.

## Claims

1. Cosmetic composition, **characterized in that** it comprises, in a cosmetically acceptable medium:
a) at least one amphoteric starch chosen from the compounds of formulae (I) to (IV): 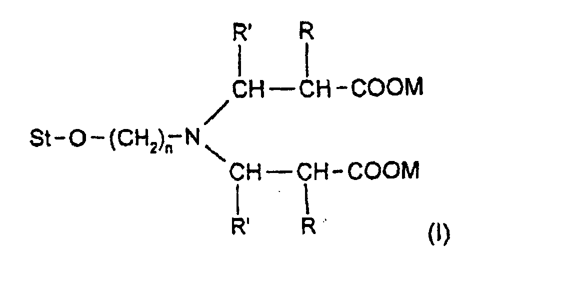 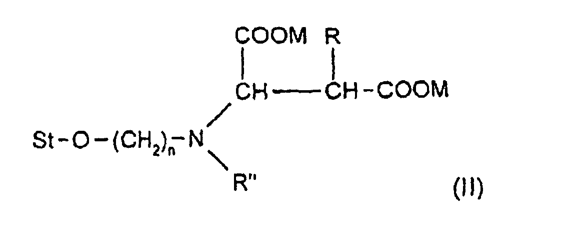 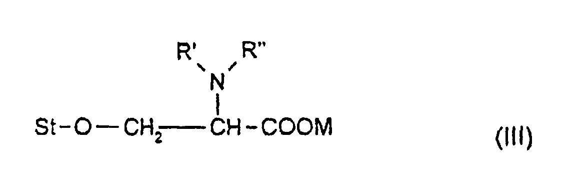 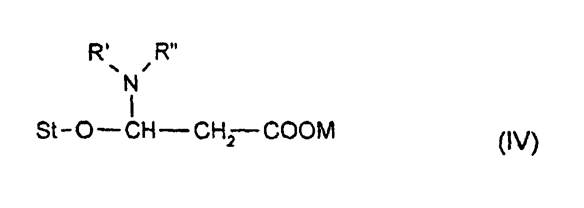 in which formulae:
St-O represents a starch molecule,
R, which may be identical or different, represents a hydrogen atom or a methyl radical,
R', which may be identical or different, represents a hydrogen atom, a methyl radical or a -COOH group,
n is an integer equal to 2 or 3,
M, which may be identical or different, denotes a hydrogen atom, an alkali metal or alkaline-earth metal, NH₄, a quaternary ammonium or an organic amine,
R" represents a hydrogen atom or an alkyl radical containing from 1 to 18 carbon atoms,
and b) at least one cationic conditioner chosen from:
- cationic silicones,
- surfactants of quaternary ammonium salt type,
- cyclopolymers of alkyldiallylamine or of dialkyldiallylammonium, and
- polyquaternary ammonium polymers chosen from:
(1) diquaternary ammonium polymers containing repeating units corresponding to formula (IV): 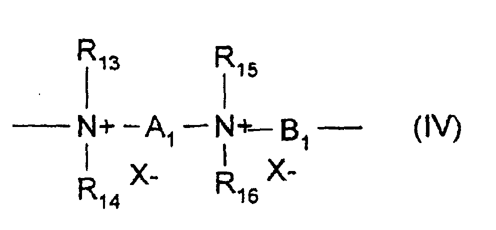 in which formula (IV):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second hetero atom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O) ₓ-CH₂-CH₂-
-[CH₂-CH (CH₃)-O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon radical, or alternatively the divalent radical
-CH₂-CH₂-S-S-CH₂-CH₂-;
d) a ureylene group of formula: -NH-CO-NH-;
(2) polyquaternary ammonium polymers consisting of units of formula (VII): 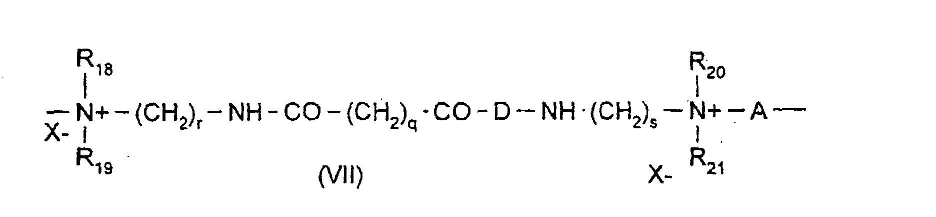 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X⁻ denotes an anion of an inorganic or organic acid, such as a halide,
D may be nothing or may represent a group -CH₂)ₜ-CO- in which t denotes a number equal to 4 or 7,
A denotes a dihalide radical or preferably represents -CH₂-CH₂-O-CH₂-CH₂-.

2. Composition according to the preceding claim, **characterized in that** the starches are of formula (I) or (II).

3. Composition according to the preceding claim, **characterized in that** R, R' and R" represent a hydrogen atom and n is equal to 2.

4. Composition according to any one of the preceding claims, **characterized in that** the aminosilicone is chosen from:
(a) the polysiloxanes referred to in the CTFA dictionary as "amodimethicone" and corresponding to the formula: 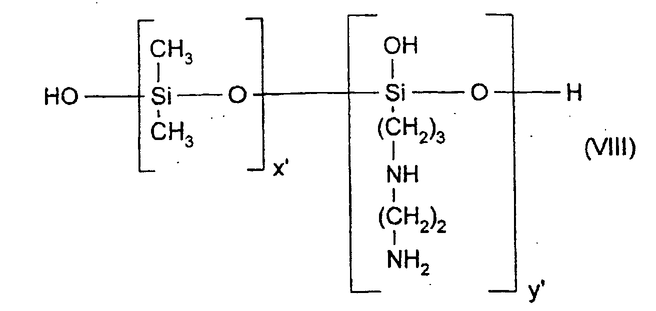 in which x' and y' are integers dependent on the molecular weight, generally such that the said number-average molecular weight is between 5 000 and 500 000;
(b) aminosilicones corresponding to the formula:
R'ₐG₃₋ₐ-Si(OSiG₂) ₙ- (OSiG_{b}R' _{2-b})ₘ-O-SiG₃₋ₐ-R'ₐ (IX)
in which:
G is a hydrogen atom or a phenyl, OH or C₁-C₈ alkyl group, for example methyl,
a denotes the number 0 or an integer from 1 to 3, in particular 0,
b denotes 0 or 1, and in particular 1,
m and n are numbers such that the sum (n + m) can range especially from 1 to 2 000 and in particular from 50 to 150, it being possible for n to denote a number from 0 to 1 999 and especially from 49 to 149 and it being possible for m to denote a number from 1 to 2 000 and especially from 1 to 10;
R' is a monovalent radical of formula -C_{q}H_{2q}L in which q is a number from 2 to 8 and L is an optionally quaternized amine group chosen from the groups:
-NR"-CH₂-CH₂-N'(R")₂
-N(R")₂
-N^{⊕}(R")₃A⁻
-NH^{⊕}(R")₂A⁻
-NH₂^{⊕}(R")A⁻
-N(R")-CH₂-CH₂-N^{⊕}R"H₂A⁻,
in which R" can denote hydrogen, phenyl, benzyl, or a saturated monovalent hydrocarbon radical, for example an alkyl radical having from 1 to 20 carbon atoms, and A⁻ represents a halide ion such as, for example, fluoride, chloride, bromide or iodide;
(c) aminosilicones corresponding to the formula: 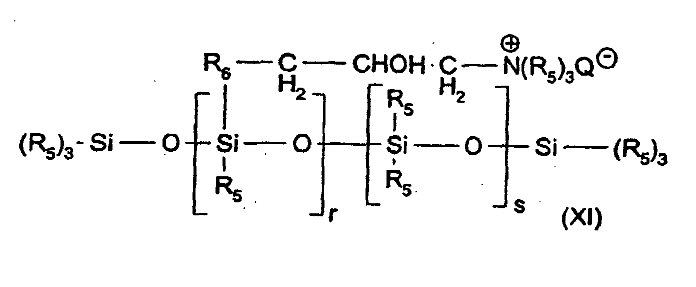 in which:
R₅ represents a monovalent hydrocarbon radical having from 1 to 18 carbon atoms, and in particular a C₁-C₁₈ alkyl or C₂-C₁₈ alkenyl radical, for example methyl;
R₆ represents a divalent hydrocarbon radical, in particular a C₁-C₁₈ alkylene radical or a divalent C₁-C₁₈, for example C₁-C₈, alkylenoxy radical connected to the Si by an SiC bond;
Q⁻ is an anion such as a halide ion, in particular chloride, or an organic acid salt;
r represents an average statistical value from 2 to 20 and in particular from 2 to 8;
s represents an average statistical value from 20 to 200 and in particular from 20 to 50;
d) quaternary ammonium silicones of formula: 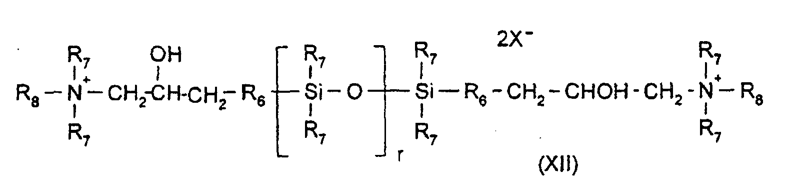 in which:
R₇, which may be identical or different, represent a monovalent hydrocarbon radical having from 1 to 18 carbon atoms, and in particular a C₁-C₁₈ alkyl radical, a C₂-C₁₈ alkenyl radical or a ring comprising 5 or 6 carbon atoms, for example methyl;
R₆ represents a divalent hydrocarbon radical, in particular a C₁-C₁₈ alkylene radical or a divalent C₁-C₁₈, for example C₁-C₈, alkylenoxy radical connected to the Si by an SiC bond;
R₈, which may be identical or different, represent a hydrogen atom, a monovalent hydrocarbon radical having from 1 to 18 carbon atoms, and in particular a C₁-C₁₈ alkyl radical, a C₂-C₁₈ alkenyl radical or a radical -R₆-NHCOR₇;
X⁻ is an anion such as a halide ion, in particular chloride, or an organic acid salt (acetate, etc.);
r represents an average statistical value from 2 to 200 and in particular from 5 to 100;
e) aminosilicones of formula (XIII): 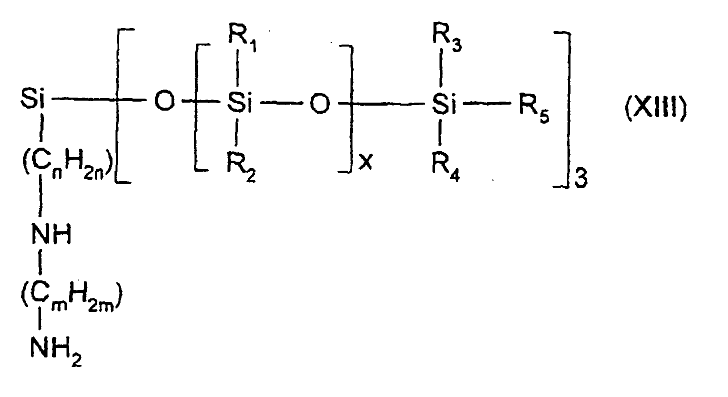 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5,
and in which x is chosen such that the amine number is between 0.01 and 1 meq/g.

5. Composition according to any one of Claims 1 to 3, **characterized in that** the surfactants of quaternary ammonium salt type are chosen from:
A) the quaternary ammonium salts of general formula (XIV) below: 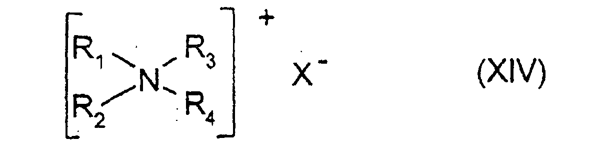 in which X is an anion chosen from the group of halides (chloride, bromide or iodide) or (C₂-C₆) alkyl sulphates, more particularly methyl sulphate, phosphates, alkyl or alkylaryl sulphonates, anions derived from organic acids, such as acetate or lactate, and
i) the radicals R₁ to R₃, which may be identical or different, represent a linear or branched aliphatic radical containing from 1 to 4 carbon atoms, or an aromatic radical such as aryl or alkylaryl. The aliphatic radicals can comprise hetero atoms such as, in particular, oxygen, nitrogen, sulphur or halogens;
R₄ denotes a linear or branched alkyl radical containing from 16 to 30 carbon atoms.
ii) the radicals R₁ and R₂, which may be identical or different, represent a linear or branched aliphatic radical containing from 1 to 4 carbon atoms, or an aromatic radical such as aryl or alkylaryl. The aliphatic radicals can comprise hetero atoms such as, in particular, oxygen, nitrogen, sulphur or halogens. The aliphatic radicals are chosen, for example, from alkyl, alkoxy, alkylamide and hydroxyalkyl radicals containing from about 1 to 4 carbon atoms;
R₃ and R₄, which may be identical or different, denote a linear or branched alkyl radical containing from 12 to 30 carbon atoms, the said radical comprising at least one ester or amide function.
R₃ and R₄ are chosen in particular from (C₁₂-C₂₂) alkylamido (C₂-C₆) alkyl and (C₁₂-C₂₂)alkylacetate radicals;
B) - quaternary ammonium salts of imidazolinium, such as, for example, that of formula (XV) below: 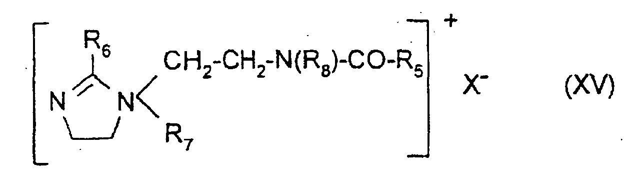
in which R₅ represents an alkenyl or alkyl radical containing from 8 to 30 carbon atoms, for example fatty acid derivatives of tallow, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, and X is an anion chosen from the group of halides, phosphates, acetates, lactates, alkyl sulphates, alkyl sulphonates or alkylaryl sulphonates;
C) - diquaternary ammonium salts of formula (XVI): 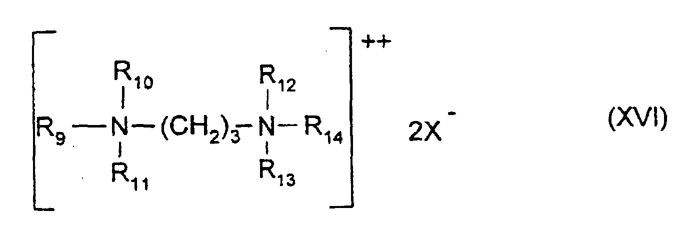 in which R₉ denotes an aliphatic radical containing from about 16 to 30 carbon atoms, R₁₀, R₁₁, R₁₂, R₁₃ and R₁₄, which may be identical or different, are chosen from hydrogen and an alkyl radical containing from 1 to 4 carbon atoms, and X⁻ is an anion chosen from the group of halides, acetates, phosphates, nitrates and methyl sulphates;
D) - quaternary ammonium salts containing at least one ester function, of formula (XVII) below: 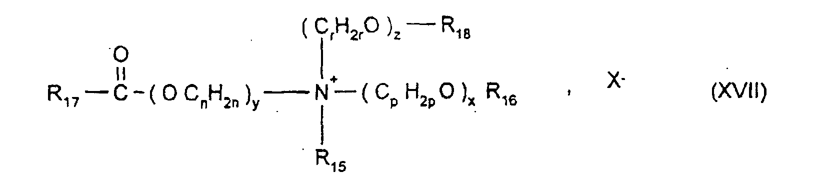
in which:
- R₁₅ is chosen from C₁-C₆ alkyl radicals and C₁-C₆ hydroxyalkyl or dihydroxyalkyl radicals;
- R₁₆ is chosen from:
- a radical 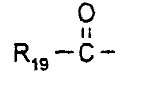
- linear or branched, saturated or unsaturated C₁-C₂₂ hydrocarbon-based radicals R₂₀,
- a hydrogen atom,
- R₁₈ is chosen from:
- a radical 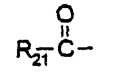
- linear or branched, saturated or unsaturated C₁-C₆ hydrocarbon-based radicals R₂₂,
- a hydrogen atom,
- R₁₇, R₁₉ and R₂₁, which may be identical or different, are chosen from linear or branched, saturated or unsaturated C₇-C₂₁ hydrocarbon-based radicals;
- n, p and r, which may be identical or different, are integers ranging from 2 to 6;
- y is an integer ranging from 1 to 10;
- x and z, which may be identical or different, are integers ranging from 0 to 10;
- X⁻ is a simple or complex, organic or inorganic anion;
with the proviso that the sum x + y + z is from 1 to 15, that when x is 0, then R₁₆ denotes R₂₀ and that when z is 0, then R₁₈ denotes R₂₂.

6. Composition according to any one of the preceding claims, **characterized in that** the said cationic surfactant is chosen from behenyltrimethylammonium salts, stearamidopropyldimethyl(myristyl acetate) ammonium salts, Quaternium-27 and Quaternium-83.

7. Composition according to any one of the preceding claims, **characterized in that** the cyclopolymers of alkyldiallylamine or of dialkyldiallylammonium are chosen from homopolymers or copolymers comprising, as main constituent of the chain, units corresponding to the formula (XVIII) or (XIX): 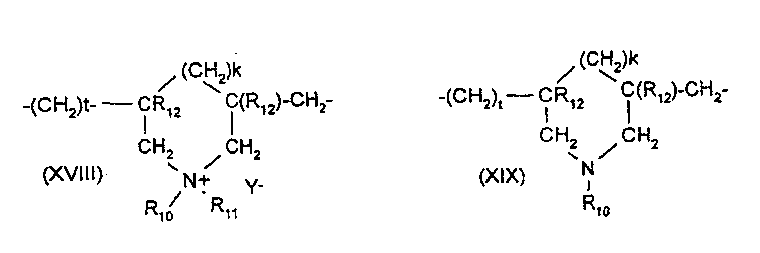 in which formulae k and t are equal to 0 or 1, the sum k + t being equal to 1; R₁₂ denotes a hydrogen atom or a methyl radical; R₁₀ and R₁₁, independently of each other, denote an alkyl group having from 1 to 22 carbon atoms, a hydroxyalkyl group in which the alkyl group preferably has 1 to 5 carbon atoms, a lower C₁-C₄ amidoalkyl group, or R₁₀ and R₁₁ can denote, together with the nitrogen atom to which they are attached, heterocyclic groups such as piperidyl or morpholinyl; Y⁻ is an anion such as bromide, chloride, acetate, borate, citrate, tartrate, bisulphate, bisulphite, sulphate or phosphate.

8. Composition according to any one of Claims 1 to 5, **characterized in that** the diquaternary ammonium polymers containing repeating units corresponding to formula (VI) consist of repeating units corresponding to the formula: 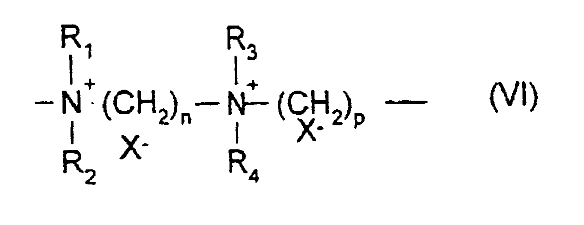 in which R₁, R₂, R₃ and R₄, which may be identical or different, denote an alkyl or hydroxyalkyl radical containing from 1 to 4 carbon atoms approximately, n and p are integers ranging from 2 to 20 approximately, and X⁻ is an anion derived from an inorganic or organic acid.

9. Composition according to any one of Claims 1 to 8, **characterized in that** the amphoteric starch is present in concentrations of between 0.01% and 10% and preferably between 0.1% and 5% by weight relative to the total weight of the composition.

10. Composition according to any one of the preceding claims, **characterized in that** the cationic conditioner is present at a concentration of between 0.001% and 10% by weight relative to the total weight of the composition, preferably between 0.01% and 5% by weight.

11. Composition according to any one of the preceding claims, **characterized in that** it also comprises at least one surfactant chosen from anionic, nonionic and amphoteric surfactants, and mixtures thereof.

12. Composition according to Claim 11, **characterized in that** the surfactant(s) is (are) present at a concentration of between 0.1% and 60% by weight, preferably between 3% and 40% by weight and even more preferably between 5% and 30% by weight, relative to the total weight of the composition.

13. Composition according to any one of Claims 1 to 12, **characterized in that** it comprises at least one additive chosen from thickeners, fragrances, nacreous agents, preserving agents, silicone or non-silicone sunscreens, vitamins, provitamins, cationic, amphoteric, anionic or nonionic polymers, proteins, protein hydrolysates, 18-methyleicosanoic acid, hydroxy acids, panthenol and volatile or non-volatile, cyclic or linear or crosslinked, modified or unmodified silicones.

14. Composition according to any one of Claims 1 to 13, **characterized in that** it has a pH of between 2 and 10.

15. Composition according to any one of Claims 1 to 14, **characterized in that** it has a pH of between 3 and 6.5.

16. Composition according to any one of the preceding claims, **characterized in that** it is in the form of a shampoo, a rinse-out or leave-in conditioner, a permanent-waving, straightening, dyeing or bleaching composition for the hair, a rinse-out composition to be applied between the two steps of a permanent-waving or hair-straightening operation, shower gels, bubble baths and make-up-removing products.

17. Use of a composition as defined in any one of the preceding claims, for washing or caring for keratin materials.

18. Process for treating keratin materials such as the hair, **characterized in that** it consists in applying to the said materials a cosmetic composition according to one of Claims 1 to 16, and then in optionally rinsing with water.
